# EUROPEAN PATENT APPLICATION

(11) **EP 4 740 853 A1**
(43) Date of publication of application: **13.05.2026**
(21) Application number: 24383221.9
(22) Date of filing: 08.11.2024
(51) Int. Cl.: A61B 5/11, A61B 5/00

(54) **METHOD, SYSTEM, AND DEVICES FOR DETECTING AND QUANTIFYING BODY MOTION**

(71) Applicant: Indivi AG, 4051 Basel (CH)
(72) Inventor: ENA BERNAD, Alejandro, 30500 Murcia (ES); MAZZÀ, Claudia, 8037 Zürich (CH); BELACHEW, Shibeshih Mitiku, 8044 Zürich (BE); REYES PUPO, Óscar Gabriel, 14011 Córdoba (ES)
(74) Representative: Vossius & Partner Patentanwälte Rechtsanwälte mbB

(57) **Abstract**

The present disclosure relates to a method for motion detection and quantification. The method comprises the steps of obtaining a time-domain data signal based on raw motion data, determining a time-frequency representation, TFR, of the data signal, determining a ridge in the TFR, wherein the ridge corresponds to locations of maximum energy or maximum amplitude in the TFR and comprises a motion-related frequency, *fa*, per each time step of the data signal, splitting the time-domain data signal into one or more stationary time step segments based on the determined ridge, reconstructing motion data based on the one or more stationary time step segments of the data signal, and determining and quantifying the motion data based on temporal characteristics of the reconstructed motion data.

## Description

### Field of disclosure

The disclosure relates to methods, systems, and devices for detecting and quantifying motion of body parts, body segments, and/or the whole body.

The disclosure relates, in particular, to methods, systems, and devices for human motion detection and quantification using a frequency-adaptive segmentation of motion data.

### Technical background

Human motion quantification is highly relevant in the assessment and monitoring of e.g. neurological or musculo-skeletal diseases that impair an individual's movement. Here, obtaining objective and measurable data about an individual's motor abilities is of paramount importance in different domains, such as gait, balance, coordination and dexterity.

As an example of human motion, gait is typically described as a cyclic movement, with the duration of individual gait cycles typically quantified by detecting (i) the interval between two subsequent initial contacts of the same foot with the ground (stride) and (ii) the interval between initial contact of one foot and next initial contact of the contralateral foot (step). Changes in these essential parameters of gait are typically first indicators of impaired motor abilities. Other examples of human motion are sit-stand transitions where an individual repeats cycles of standing and sitting, and alternate pronation-supination movements such as, e.g., rotating the forearm back and forth between a palm down orientation and a palm up orientation.

Gait impairments are commonly quantified through observational gait analysis and quantitative gait analysis. Observational gait analysis techniques are subject to bias and have limited accuracy [1]. On the other hand, quantitative gait analysis techniques seek to eliminate possible human interpretation bias [2, 3] by employing technologies such, e.g., as pressure-sensitive walkways and motion capture systems. However, these technologies are expensive and require a laboratory environment, which limits their use to very infrequent and supervised assessments of an individual's gait.

The increased prevalence and technological advancement of modern mobile and wearable devices in recent years has led to new techniques and technologies that aim to leverage sensors typically provided in these modern devices such as, e.g., digital cameras, microphones, global positioning systems (GPS), and inertial measurement units (IMUs - accelerometers, gyroscopes, magnetometers). These new techniques and technologies aim to provide affordable, and accurate monitoring tools [4, 5] for both clinical use and home-based monitoring.

In conjunction, algorithms for quantification of sensor-based measurements from the IMU data [6] have been proposed. However, when applied to IMU data from mobile devices, such as smartphones, and unsupervised data collection conditions, the accuracy of measurements is heavily affected by lower quality of the electronics, movement artifacts, and by the varying orientation and positioning of the devices relative to an individual's body. The varying severity of neurological impairments, causing irregular or jerky movements, further exacerbate these issues. Overall, even basic gait analysis remains highly challenging because of suboptimal accuracy of step detection and segmentation in relation to the aforementioned technical and physiological sources of variability. Simple methods such as thresholding [7] or zero-crossing detection [8] have been proposed that are easy to implement, but face challenges with individuals with abnormal gait patterns, and advanced methods utilizing deep learning [9] may offer higher accuracy but are difficult to implement as they require substantial computational resources and availability of large, labeled datasets.

Overall, main drawbacks of the state-of-the-art can be summarized as:
- High dependence on device location: For example, algorithms designed for data located near the body's Center of Mass, e.g., L5 - 5th lumbar vertebrae, cannot be readily translated to a device worn in another body location like a clothing pocket such as, e.g., a pants pocket, as is commonly the case for an approach utilizing smartphones.
- Required assumption of a steady walking cadence: This leads to significant challenges when applied to individuals with neurological diseases who exhibit high variability in their gait patterns that becomes even more pronounced in daily-life environments.
- Limited accuracy in the presence of noise and artifact: Robust signal pre-processing algorithms are lacking. For example, filters at fixed frequencies are often used for the entire data signal, meaning that not all the background noise is removed. This can lead to an insufficient signal-to-noise ratio.

There is therefore a need to address the above issues of the state-of-the art.

### Summary of the disclosure

Some or all issues of the state-of-the-art are addressed by the disclosure as defined by the independent claims. Preferred embodiments are defined by the sub-features of the dependent claims.

In an aspect, the present disclosure relates to a method for motion detection and quantification. The method comprises the steps of:
a) obtaining a time-domain data signal based on raw motion data;
b) determining a time-frequency representation, TFR, of the data signal;
c) determining a ridge in the TFR, wherein the ridge corresponds to locations of maximum energy or maximum amplitude in the TFR and comprises a motion-related frequency, *fa,* per each time step of the data signal;
d) splitting the time-domain data signal into one or more stationary time step segments based on the determined ridge;
e) reconstructing motion data based on the one or more stationary time step segments of the data signal; and
f) determining and quantifying the motion data based on temporal characteristics of the reconstructed motion data.

By reconstructing motion data from stationary time step segments of the time-domain signal determined based on the ridge, the method of the disclosure allows to dynamically extract motion-related frequencies and accurately detect and quantify different types of motion of body parts, body segments, or the whole body. The method is agnostic to the manner in which raw motion data is obtained from an individual during motion. This is, in particular, advantageous as raw motion data and motion-related frequencies in the raw motion data are dependent on and can change substantially with the type of motion to be detected and quantified.

Various embodiments may preferably implement the following features.

Preferably, in a), the raw motion data is obtained from one or more inertial measurement unit, IMU, sensors which preferably comprise one or more of an accelerometer and/or a gyroscope. These sensors are readily available, often as part of modern mobile or wearable devices, and are particularly advantageous for obtaining raw motion data for motion detection and quantification outside of a supervised laboratory environment.

Preferably, in b), determining the TFR comprises performing synchro-squeezing transform, SST, multi-synchro-squeezing transform, MSST, short-time Fourier transform, STFT, Hilbert-Huang transform, HHT, or Wigner-Ville-Distribution, WVD, on the time-domain data signal.

For human motion that provides more stationary signals, e.g., sit/stand transition motion, STFT is advantageous as it is computationally efficient and simply to implement. For human motion that provides more complex signals with time-variant frequencies, SST provides improved resolution and resilience to noise in obtaining the TFR.

Preferably, in c), determining the ridge comprises splitting the time-domain data signal and the TFR into a plurality of overlapping time step windows, and obtaining a dominant frequency, *ff*, for each time step window.

The overlap between the time step windows in the TFR leads to increased sampling of the time-domain data signal resulting in an increased temporal resolution. Additionally, due to the overlap, abrupt changes between adjacent time step windows are smoothened, thus reducing the impact of artifacts that are not representative of characteristics of the time-domain data signal and improving the signal-to-noise ratio. The increased sampling of the time-domain data also results in improved time-frequency resolution.

Preferably, obtaining *ff* for each time step window comprises determining a main frequency, *fa,* using autocorrelation on the respective time-domain data signal time step window, and correcting *f0* by determining the closest dominant frequency in the frequency spectrum of the corresponding TFR time step window.

Correcting *f0* by the closest dominant frequency provides an improved determination of the motion-related frequency *f0* comprised by the ridge, as the accuracy for determining the main frequency *f0* can be affected by noise and artifacts.

Preferably, in c),fa is obtained based on the obtained *ff* values of the time step windows.

Preferably, in c), for each respective time step, *fa* is obtained by averaging the *ff* values of all the time step windows that contain the respective time step.

The foregoing two features allow for further reducing the impact of noise (related for instance to the dynamic variability of the underlying nature of the motion of interest) present for determining the ridge.

Preferably, each time step window has a constant window time step size n.

This reduces requirements for computational resources.

Preferably, each time step window overlaps adjacent time step windows by n minus m time steps, wherein m<n, and wherein m equals 3, preferably m equals 2, more preferably m equals 1.

Preferably, each time step window overlaps adjacent time step windows by more than 70%, preferably by more than 80%, more preferably by more than 90%, even more preferably by more than 95% of the window time step size.

The foregoing two features allow adjustment of the temporal resolution of the ridge in a simple and computationally efficient manner.

Preferably, in d), splitting the time-domain data signal into stationary time step segments comprises determining stationary time step segments based on one or more jumps in the ridge that correspond to changes in the tendency of the ridge, in the time-frequency-plane, that exceed a predetermined frequency threshold, and splitting the time domain data signal into stationary time step segments separated by respective time steps where the one or more jumps in the ridge are determined.

Within the context of the present disclosure, a stationary time step segment is a segment within which one or more statistical properties such as, e.g., mean, variance, autocorrelation, of the time-domain data signal does not substantially change.

In this way, for a stationary time step segment of the time-domain data signal, the corresponding ridge has a limited spectral bandwidth of two times the frequency threshold since deviations to higher or lower frequencies up to the frequency threshold are allowed. This means that the motion-related frequency *fa* for the stationary time step segment is confined within the limited spectral bandwidth. In other words, the motion/pattern underlying the stationary time step segment does not substantially change.

Preferably, the predetermined frequency threshold is in a range from 0.05 Hz to 0.2 Hz, preferably from 0.07 to 0.13 Hz, most preferably about 0.1 Hz.

These frequency thresholds have shown to be optimal at determining jumps in the ridge, and thus have shown to provide optimal splitting of the time-domain data signal into stationary time step segments. If the threshold is too low, the time-domain data signal will be split into too many segments, thus complicating reconstructing motion data. Conversely, if the threshold is too high, stationary time step segments of the time-domain data signal will not be substantially stationary, as the motion-related frequency *fa* undergoes larger changes within the stationary time step segment due to the higher frequency threshold.

Preferably, in e), reconstructing the motion data comprises, for each stationary time step segment, determining a mean main frequency,*fs*, of a respective stationary time step segment by averaging *fa* values of the respective segment of the ridge that corresponds to the time steps of the respective stationary time step segment, determining a frequency spectrum of the respective segment of the TFR, determining upper and lower bounds for the band frequency around *fs*, and filtering each respective stationary time step segment using a bandpass filter.

This allows reconstructing motion data from the time-domain signal by extracting the frequency bands of the stationary segments that are most indicative of the type of motion to be detected and quantified.

Preferably, in c), determining the ridge further comprises removing a first portion of time steps at the beginning and end of the time-domain data signal and TFR. Additionally, in e), reconstructing the motion data further comprises reconstructing the removed first portion of total time steps at the beginning and end of the ridge, using the mean frequencies of a second portion of time steps of the ridge respectively after and before the removed first portion of total time steps to be reconstructed.

This allows to account for edge effects when obtaining a TFR of the time-domain data signal that cause distortions and inaccuracies in the TFR. Additionally, this allows to account for non-stationary motion at the beginning and/or end of a measured movement. For example, when measuring cadence, this allows to account for an initial acceleration to normal cadence at the beginning of the measurement, and for a final deceleration at the end of the measurement.

Preferably, in e), reconstructing the motion data further comprises reconstructing a final filtered signal based on the respective filtered stationary time step segments.

Preferably, in e), determining and quantifying the motion data comprises determining motion events based on one or more of local extrema in the reconstructed motion data, zero-crossing in the reconstructed motion data, an amplitude of the reconstructed motion data, and/or a distance between local extrema based on frequencies comprised by the ridge.

Preferably, in a), obtaining the time-domain data signal further comprises pre-processing the raw motion data by one or more of rotating the signals to a global reference frame, applying a filter to remove noise, and/or applying a filter to keep band of frequencies associated with the motion to be detected and quantified.

This simplifies the overall detection and quantification process. Rotating to a global reference standardizes and simplifies processing of the obtained raw motion data, while filtering to keep frequencies associated with the motion to be detected and quantified reduces the amount of data to be processed to further simplify the processing of the obtained raw motion data.

In a further aspect, the present disclosure relates to an apparatus comprising at least one processor configured to perform a method according to any one of the above-specified methods and respective embodiments.

Preferably, the apparatus comprises or substantially consists of a mobile computing device.

This improves detection and quantification of human motion for the present disclosure as a mobile computing device, such as, e.g., a smartphone, smartwatch or other mobile and/or wearable device allows to collect raw motion data outside of an artificial and supervised laboratory environment and over prolonged time periods during an individual's daily activities.

In a further aspect, the present disclosure relates to a computer program product comprising instructions that, when executed by an apparatus comprising at least one processor, cause the apparatus to perform a method according to any one of the above-specified methods and respective embodiments.

In a further aspect, the present disclosure relates to a computer-readable storage medium comprising the computer program product according to the aspect.

Other aspects, features, and advantages will be apparent from the summary above, as well as from the description that follows, including the figures and the claims.

Embodiments of the present disclosure will now be described by way of example only and with reference to the following accompanying drawings. In the figures, the same reference numerals denote the same or similar elements.

### Brief description of the drawings

Fig. 1 shows a flow diagram illustrating a method for determining and quantifying human motion according to embodiments of the present disclosure.
Fig. 2 shows a flow diagram illustrating a method for determining and quantifying human gait according to embodiments of the present disclosure.
Fig. 3 shows a flow diagram illustrating a method for determining and quantifying human gait according to further embodiments of the present disclosure.
Fig. 4 shows a schematic illustration of raw motion data signal overlayed with a pre-processed signal according to embodiments of the present disclosure.
Figs. 5 and 6 respectively show a schematic illustration of an obtained TFR and frequency spectrum of the time-domain data signal for the embodiment of Fig. 4.
Fig. 7 shows a schematic illustration of a frequency spectrum for an exemplary time step window for the embodiment of Fig. 4.
Fig. 8 shows a schematic illustration of the TFR shown in Fig. 5 with a determined ridge as an overlay.
Fig. 9 shows a schematic illustration of a plot for determining stationary segments according to embodiments of the present disclosure.
Fig. 10 shows a schematic illustration of a frequency spectrum of an exemplary stationary time step segment of the time-domain data signal for the embodiment of Fig. 4.
Fig. 11 shows a schematic illustration of the time-domain data signal for the embodiment of Fig. 4 overlayed with the respectively obtained final filtered signal for step segmentation.
Fig. 12 shows a schematic illustration of the overlaid final filtered signal of Fig. 11 for step segmentation.
Figs. 13A to 13D show schematic illustrations of respective plots for detecting and quantifying human motion according to embodiments of the present disclosure.
Figs. 14A to 14D show schematic illustrations of respective plots for detecting and quantifying human motion according to further embodiments of the present disclosure.
Figs. 15A to 15D show schematic illustrations of respective plots for detecting and quantifying human motion according to embodiments of the present disclosure.
Figs. 16A to 15D show schematic illustration of plots showing comparative performance for gait detection according to further embodiments of the present disclosure.
Figs. 17A to 17C show schematic illustration of plots showing comparative performance for gait detection according to yet further embodiments of the present disclosure.
Figs. 18A to 18C show schematic illustration of plots showing comparative performance for gait detection according to yet further embodiments of the present disclosure.
Figs. 19 to 19C show schematic illustration of plots showing comparative performance for gait detection according to yet further embodiments of the present disclosure.
Fig. 20 shows schematic illustrations of obtained time-domain data signals overlayed with respective final filtered signals according to further embodiments of the present disclosure.
Figs. 21A and 21B show schematic illustrations of obtained time-domain data signals overlayed with respective final filtered signals according to yet further embodiments of the present disclosure.

### Detailed description

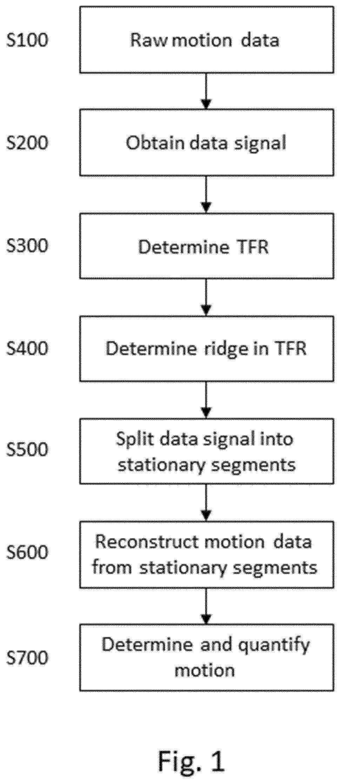

**Fig. 1** shows a flow diagram illustrating a method for detecting and quantifying human motion, according to embodiments of the present disclosure.

In step **S100**, raw motion data is obtained. The raw motion may comprise or substantially consist of pre-recorded data that is merely made available in step S100. Additionally, or alternatively, the raw motion may comprise or substantially consists of data obtained from any suitable device such as, e.g., sensors.

In step **S200,** a time-domain data signal is obtained from the raw motion data. Depending on the type, format or other relevant characteristic of the raw motion data, the raw motion data may be further transformed to obtain a time-domain data signal.

In step **S300,** a time-frequency representation, TFR, of the time-domain data signal is obtained. In principle, any suitable process known in the art for obtaining a TFR can be used. Utilizing the TFR of the obtained time-domain data signal allows to gain insights on the frequency contents of the time-domain data signal and its evolution over time, which is useful for analyzing a time-domain data signal obtained from an individual with motion impairment, as their motion behavior may differ significantly from the motion behavior to be expected from a healthy individual.

In step **S400,** based on the obtained TFR, a ridge in the TFR is determined. The ridge in the TFR corresponds to a path or curve that tracks locations, in the time-frequency plane of the TFR, that correspond to locations of maximum energy or amplitude of the time-domain data signal. These locations correspond to motion-related frequencies *fa,* i.e., frequencies in the time-domain data signal most indicative of a certain motion to be detected. Notably, the approach for determining the ridge is dependent on the type of motion to be detected and quantified. Embodiments of the present disclosure allow to determine the ridge by dynamically selecting motion-related frequencies, as will be detailed in the context of embodiments described below.

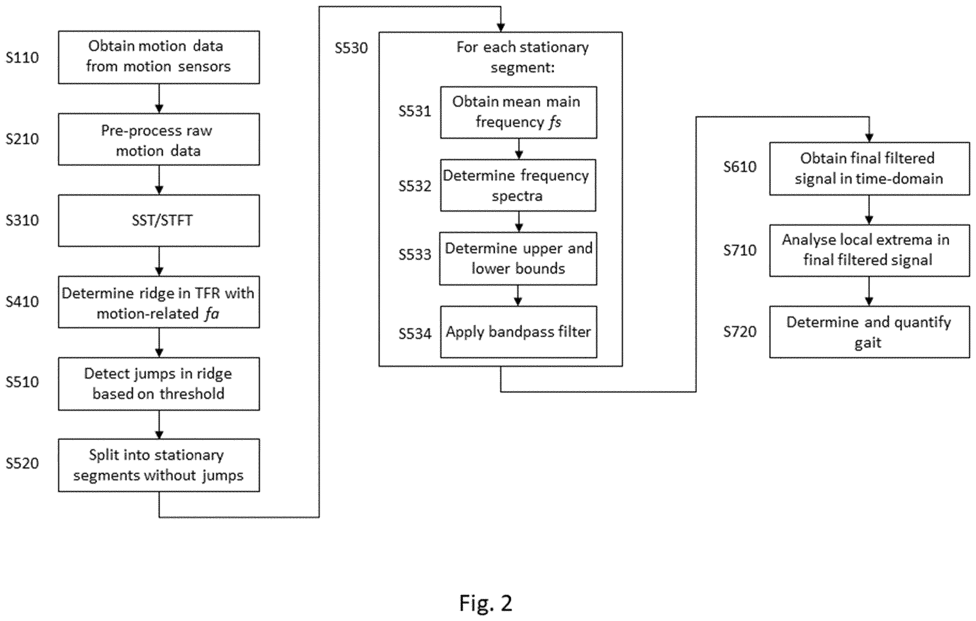

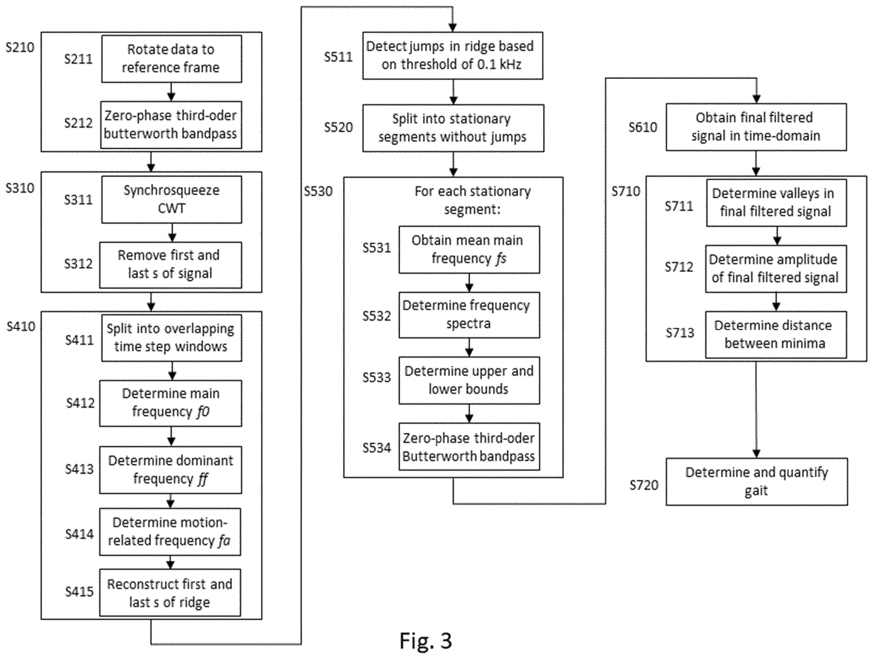

In step **S500,** based on the determined ridge in the TFR, the time-domain data signal can be split into stationary time step segments. Notably, stationary time step segments of the time-domain data signal may be separated from each other at certain time steps based on the spectra-temporal characteristics of the ridge at these certain time steps. For example, stationary time step segments of time-domain data signal may be separated by time steps where the ridge exhibits changes that meet predefined criteria, as will be further detailed in the context of embodiments illustrated in Figs. 2 and 3.

In step **S600,** motion data is reconstructed from the stationary time step segments of the time-domain data signal. The stationary time step segments correspond to segments within which one or more statistical properties such as, e.g., mean, variance, autocorrelation, of the time-domain data signal do not substantially change, and thus correspond to segments of the time-domain data signal that are the least affected by noise, artefacts and irregularities in the time-domain data signal. The motion data is thus reconstructed from segments of the time-domain data signal within which the underlying movement does not substantially change along with time.

In step **S700,** the reconstructed motion data can be analyzed to detect and quantify the individual's motion, wherein the type of analysis to be performed depends on the type of motion to be detected or quantified.

**Fig. 2** shows a flow diagram illustrating a method for detecting and quantifying human motion that comprises further modifications to the method described above in the context of embodiments illustrated in Fig.1.

Step **S100** as described above within the context of Fig.1 may comprise a step **S110** of obtaining raw motion from one or more motion sensors. The one or more motion sensors are preferably attached to or otherwise associated with an individual's body. For example, accelerometers or gyroscopes can be provided to an individual as dedicated sensors. In embodiments, raw motion data is obtained from one or more inertial measurement units, IMUs, that combine a plurality of accelerometers, gyroscopes, and/or magnetometers in a single unit. Most advantageously, according to embodiments of the disclosure, the raw motion data is obtained from a mobile device like a smartphone or wearable device like a smartwatch that can be carried or worn by the individual during a substantial portion of the day during daily activities. This is in contrast to traditional methods for detecting and quantifying certain types of motion in supervised laboratory environments, where data is obtained only for limited time periods at limited intervals where the individual may exhibit behavior that is different from their behavior during daily activities.

**Step S200** as described above within the context of Fig.1 may comprise a step **S210** of performing pre-processing on the raw motion data to obtain a time-domain data signal. For example, the raw motion data may comprise or substantially consist of raw acceleration data that is pre-processed to obtain the time-domain data signal. Pre-processing may comprise one or more of filtering noise, rotating and/or normalizing the obtained raw motion data.

Step **S300** of determining the TFR of the time-domain data signal as described above within the context of Fig. 1 may comprise a **S310** of performing synchro-squeezing transform, SST, multi-synchro-squeezing transform, MSST, short-time Fourier transform, STFT, Hilbert-Huang transform, HHT, Wigner-Ville-Distribution, WVD, or short time Fourier transform, STFT, or any other similar and suitable method on the time-domain data signal. The preferred method to obtain the time-frequency representation from a time-domain data signal depends on the type of motion to be detected, and on the complexity of the associated raw motion data.

STFT is a Fourier transform that can be used to determine the time-variant spectral content and phase of local segments of the time-domain data signal as it changes overtime. In contrast to standard Fourier-transform, determining the STFT comprises dividing the time-domain data signal into shorter segments of equal length and then determining the Fourier transform separately on each shorter segment. By plotting the changing spectra determined for each shorter segment as a function of time, a TFR known as a spectrogram is obtained. As an alternative, continuous wavelet transform, CWT, can be performed to obtain the TFR. In CWT, the time-domain data signal is convolved with scaled and shifted versions of a wavelet function - a small oscillatory waveform with a specific frequency and shape. By scaling the wavelet function, its frequency can be adjusted. By shifting the wavelet function, it can be localized at different time steps. At each scale and shift, the respective wavelet function is multiplied/integrated with the time-domain data signal, resulting in coefficients representing how much the wavelet function resembles the time-domain data signal at that scale and time step. By collecting the coefficients, the TFR known as scalogram is obtained. Synchrosqueezing transform, SST, represent a further process on an obtained TFR. Here, the TFR can first be obtained using any suitable process, wherein CWT or STFT are preferred. Subsequently, for each point or small region of points in the TFR, a "correct" instantaneous frequency is estimated based on the time-derivative of the phase of the TFR. Subsequently, points or small regions of points that with an estimated instantaneous frequency are then reassigned or squeezed to a position in the TFR corresponding to the estimated instantaneous frequency, thus "sharpening" the TFR which leads to improved temporal resolution.

Step **S400** of determining a ridge in the TFR as described above within the context of Fig. 1 may comprise a **S410** of determining a ridge that contains the motion-related frequency, *fa,* for each time step of the time-domain data signal. Generally, the ridge corresponds to the path or curve that tracks the locations, in the time-frequency plane, of maximum energy or amplitude of the time-domain data signal. The tracked locations of the ridge correspond to the motion-related frequencies *fa* of the time-domain data signal, and thus provide a way to determine the most prominent frequency components of the time-domain data signal over time. The motion-related frequencies *fa* thus correspond to frequency components of the time-domain data signal that directly reflects the human motion to be detected. Generally,*fa* can be obtained based on main frequency, *fa,* components and dominant frequency, *ff,* components of the TFR. An exemplary approach will be described below within the context of Fig. 3.

Step **S500** of splitting the time-domain data signal into stationary time step segments, as described above within the context of Fig. 1, may further comprise steps **S510** to **S534.** In step **S510,** jumps in the ridge of the correspondingly obtained TFR are determined. Jumps in the ridge can be determined using an iterative approach. First, at an initial time step, an initial frequency of the ridge is determined. Then, the frequency of the ridge at each subsequent time step is determined and compared to the initial frequency. A jump is determined at a subsequent time step whenever the frequency difference to the initial frequency first exceeds the pre-defined frequency threshold. This subsequent time step is then set as the new initial time step with a new initial frequency, and the process of comparing the frequency of each subsequent time step of the ridge is repeated until the final time step of the ridge is reached.

In step **S520**, the time-domain data signal is split into stationary time step segments separated at time steps where the ridge exhibits jumps to ensure that the motion-related frequency *fa* does not substantially change within the stationary time step segment of the time-domain data signal. In step **S530** further comprising steps S531 to S534, each stationary time step segment is processed. Here, in **step S531,** a mean main frequency, *fs*, is obtained for each stationary time step segment of the time-domain data signal by averaging all *fa* values of the corresponding segment of the ridge. The main frequency *fs* corresponds to the frequency of the time-domain data signal where its energy is concentrated. In step S532, a frequency spectrum is obtained for each stationary time step segment of the time-domain data signal. Subsequently, upper bounds, *fsu*, and lower bounds, *fsl*, are determined based on *fs* to select a band of frequencies that is related to the type of human motion to be detected and quantified. In a subsequent step **S534**, a bandpass filter may be applied to further reduce the effect of noise and filter out unwanted frequencies.

Step **S600** of reconstructing motion data from the stationary time step segments of the time-domain data signal as described above within the context of Fig. 1 may comprise a step **S610** of obtaining a final filtered signal from the reconstructed motion data in the time-domain. The final filtered signal of the reconstructed motion data in the time-domain may be obtained by interpolating the motion data at time steps between stationary time step segments of the time-domain data signal.

Step **S700** of reconstructing motion data from the stationary time step segments of the time-domain data signal as described above within the context of Fig. 1 may comprise a step **S710** of determining one or more local extrema in the reconstructed motion data for determining and quantifying the type of human motion. A distance between local extrema may also be determined. Additionally, or alternatively, the amplitude of the final filtered signal may be determined. In step **S720**, information obtained via analysis of, e.g., local extrema and/or signal amplitude(s) may be utilized to perform gait detection.

**Fig. 3** shows a flow diagram illustrating a method for detecting and quantifying gait as an example of human motion, wherein the illustrated method comprises further modifications to the method described above within the context of embodiments illustrated in Fig. 2.

Step **S210** of pre-processing the obtained raw motion data as described within the context of Fig. 2 may comprise steps **S211** and **S212.** In step **S211,** pre-processing may comprise, depending on the sensor type, rotating and aligning the raw motion data from a local reference frame to a global reference frame. For example, in case of raw motion data obtained from a mobile device or wearable device, the local reference frame depends on the orientation of the mobile device or wearable relative to the individual whose motion is to be detected and quantified. Rotating and aligning raw motion data to a global reference frame based, e.g., on gravity directions, facilitates determining directions in the raw motion data related to human motion. For example, directions such as "up", "down", "forward", or "backwards" can be defined within the context of a global reference frame based on gravity directions, wherein the direction of gravity is "down", the opposite direction is up, and "forward" and "backward" directions can be defined to be perpendicular to the gravity direction. As an example, the alignment to the global reference frame can be performed using quaternions or gravity acceleration vectors. In step **S212,** a bandpass filter such as a zero-phase third-degree Butterworth bandpass filter may be applied. The zero-phase third-degree aspect is particularly advantageous as it provides a flat response within the passband and a smooth transition from passband to stopband, thus introducing minimal to no phase distortion to raw motion data. Additionally, the zero-phase aspect of the filter removes drift that can be caused when applying a filter. The passband may be set to filter out frequencies that are generally not related to human motion and/or to a specific type of human motion, which can be based on heuristics.

Step **S310** of performing SST or STFT for obtaining the TFR as described in the context of Fig. 2 may comprise steps **S311** and **S312.** In step **S311**, an SST on a previous CWT is performed. Subsequently, a portion of time steps at the beginning and at the end of the time-domain data signal and of the TFR are removed prior to further processing the obtained TFR. Removing portions at the beginning and end allows to address edge effects and artifacts typically associated with CWT and SST. The removed portions at the beginning and end may correspond to 1 to 10% preferably to 1 to 5 %, more preferably to 1 to 2% of the time steps of the time-domain data signal and of the TFR. In embodiments, 1s or 2s at the beginning and at the end are removed.

Step **S410** of determining the ridge comprising motion-related frequencies *fa* may further comprise steps **S411** to **S415.** In step **S411,** the time-domain data signal and the TFR are split into overlapping time step windows. The window time steps size n of one or more, preferably all overlapping time step windows may be the same and constant for all overlapping time step windows. The overlap between adjacent time step windows may generally be chosen based on available computational resources and the complexity of the type of motion to be detected and quantified. As the overlapping time step windows provide increased sampling of the time-domain data signal, the overlap between adjacent time step windows can be adjusted according to a desired amount of sampling. In embodiments, each time step window overlaps adjacent time step windows by more than 70%, preferably by more than 80%, more preferably by more than 90%, even more preferably by more than 95% of the window time step size. In embodiments, this can correspond into an overlap between adjacent time step windows of n minus m time steps, wherein m<n, and wherein m equals 3, preferably m equals 2, more preferably m equals 1. For example, in embodiments, the window time step size is set at around 5s, and the overlap between adjacent time step windows is set at around 4.98s. In step S412, a main frequency *f0* is determined for each overlapping time step window by using autocorrelation on each respective time step window of the time-domain data signal.

While autocorrelation is effective for detecting the fundamental frequency in a periodic signal, it is sensitive to noise, which is true for non-stationary data signals, in particular where the frequency content changes over time. Hence, in step **S413,** *f0* determined for each time-domain data signal time step window may be corrected using (replaced by) the closest determined dominant frequency *ff* in the frequency spectrum of each corresponding TFR time step window.

In step **S414**, motion-related frequencies *fa* comprised by the ridge in the TFR are obtained by averaging, for each respective time step, the *ff* values of each overlapping time step window that contains the respective time step.

Subsequently, in step **S415**, the portions at the beginning and at the end of the TFR removed in step **S312** are reconstructed for the ridge. This can be achieved, for example, by using the mean frequencies of portions respectively succeeding or preceding the removed portions at the beginning and end of the TFR.

Step **S510** of splitting the time-domain data signal into stationary time step segments based on jumps in the ridge as described in the context of Fig. 2 may be performed using a predetermined frequency threshold. For example, a jump in the ridge may be determined if a frequency change in the ridge exceeds a frequency threshold of 0.05 Hz to 0.2 Hz, preferably from 0.07 to 0.13 Hz, most preferably about 0.1 Hz.

Step **S520,** step **SS30** comprising steps **S531** to **S534**, step **S610** may be the same as described within the context of Fig. 2.

Step **S710** of analyzing local extrema in the final filtered signal as described within the context of Fig. 2 may comprise steps **S711** to **S713.** For example, for gait detection, in step **S711,** valleys in the final filtered signal that are indicative of foot-ground contact are determined. In step **S712,** one or more amplitudes of the final filtered signal at the valleys may be obtained. In step **S713,** temporal distances between valleys may be obtained. The frequency corresponding to the temporal distances between valleys may further be compared to motion-related frequencies *fa* comprised by the ridge. For example, if the temporal distance between two valleys is larger than the temporal distance expected based on *fa,* then it can be determined that a further foot-ground contact should be determined.

Step **S720** may be the same as described within the context of Fig. 2.

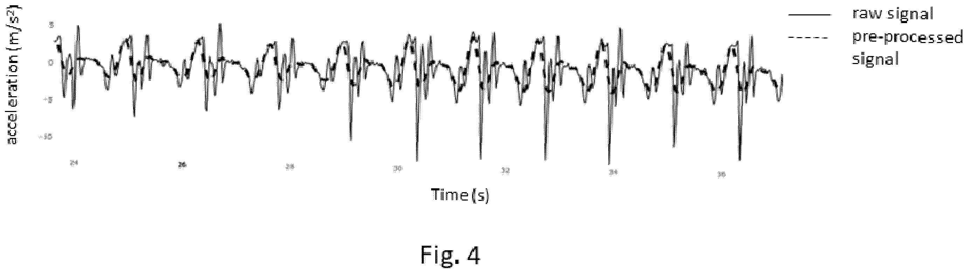

In the following, an exemplary case for a method for gait detection, as described above within the context of Figs. 2 and 3, is described for illustrative purposes within the context of **Figs. 4 to 12****.**

**Fig. 4** shows a plot illustrating raw acceleration data obtained from a smartphone (steps **S100** and **S110),** carried by an individual in a pants front pocket, as indicated by the solid lines, and a time-domain data signal indicated by the dashed line that was obtained by performing pre-processing (step **S210)** on the raw motion data. Here, a bandpass filter is applied (step **S212)** to the raw acceleration data to filter out frequencies generally unrelated to human motion.

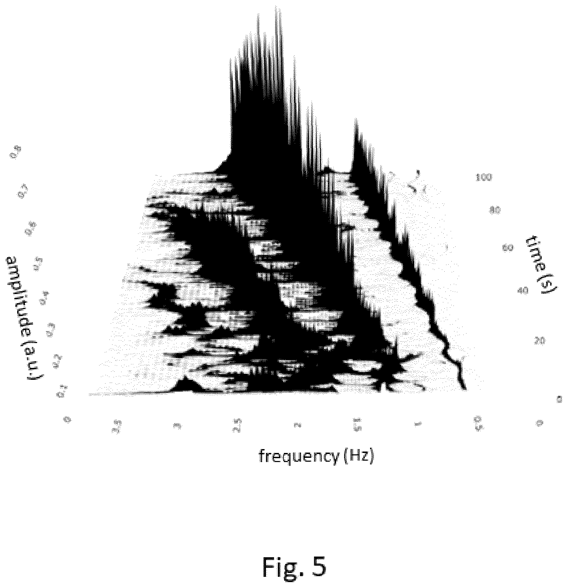

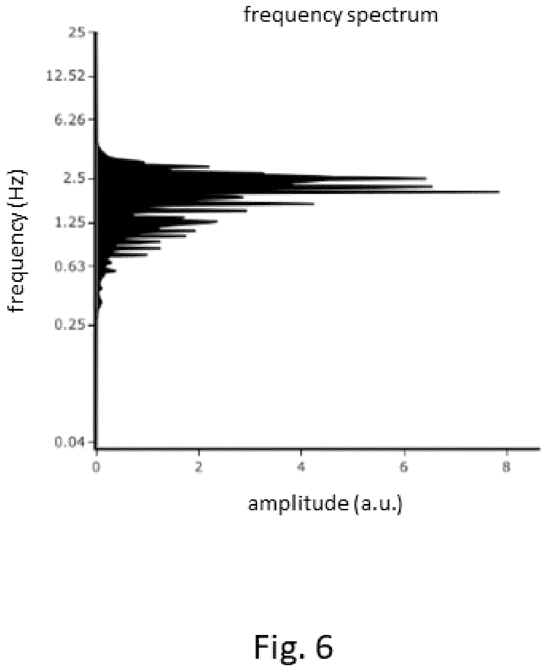

**Fig. 5** shows a TFR (step **S300)** of the time-domain data signal illustrated in Fig. 4. Here, the TFR is obtained using SSTon a CWT of the time-domain data signal (step **S310**). **Fig. 6** illustrates the frequency spectrum for the TFR.

To obtain the ridge (step **S400** and **S410),** the time-domain data signal and the TFR are split into overlapping time step windows. Here, merely for illustrative purposes, the overlapping time step windows are set to have a constant time step window size of around 5 s, with an overlap of around 4.98 s between adjacent time step windows (step S411).

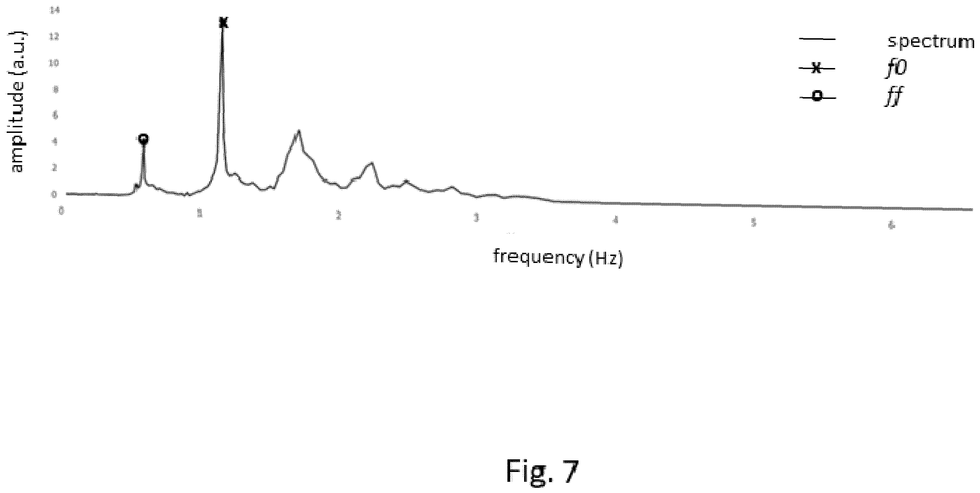

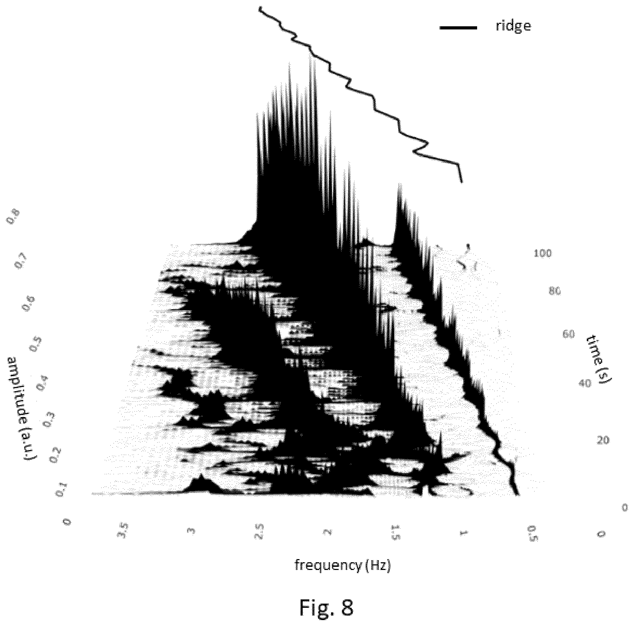

Subsequently, for each overlapping time step window, the main frequency *f0* (step **S412)** and the closest dominant frequency *ff* (step **S413)** are determined. **Fig. 7** illustrates a plot showing *f0* and *ff* for an exemplary time step window obtained from the time-domain data signal of Fig. 4 and the TFR of Fig. 5. Here, the solid lines represent the frequency spectrum for that time window, while the positions of *f0* ad *ff* are respectively denoted by "x" and "o". Here,*f0* is obtained via autocorrelation and is subsequently replaced by the closest dominant frequency *ff*. Motion related-frequencies *fa* for each respective time step are obtained by averaging *ff* values (step **S414)** of all time step windows that contain the respective time step. As a result, the ridge in the TFR as schematically illustrated in **Fig. 8** is obtained. As can be seen in Fig. 8, the ridge tracks the path or curve of locations, in the time-frequency plane of the TFR, that correspond to locations of maximum energy or amplitude of the time-domain data signal.

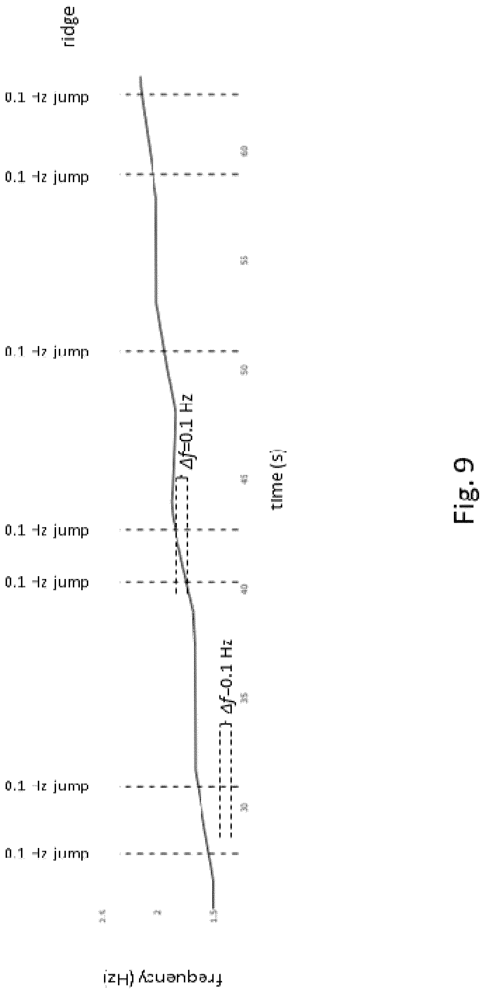

Fig. 9 shows a schematic illustration of a portion of the ridge in the time-frequency-plane. For splitting the time-domain data signal into stationary time step segments (step **S500),** jumps in the ridge are determined (step **S510).** In the case illustrated in Fig. 9, a jump is determined when the frequency of the ridge shifts by more than a frequency threshold set at 0.1 Hz. Subsequently, the time-domain data signal is split into stationary time step segments within which the motion-related frequency *fa* does not change to higher or lower frequencies by more than 0.1 Hz, i.e., does not substantially change (step **S520).**

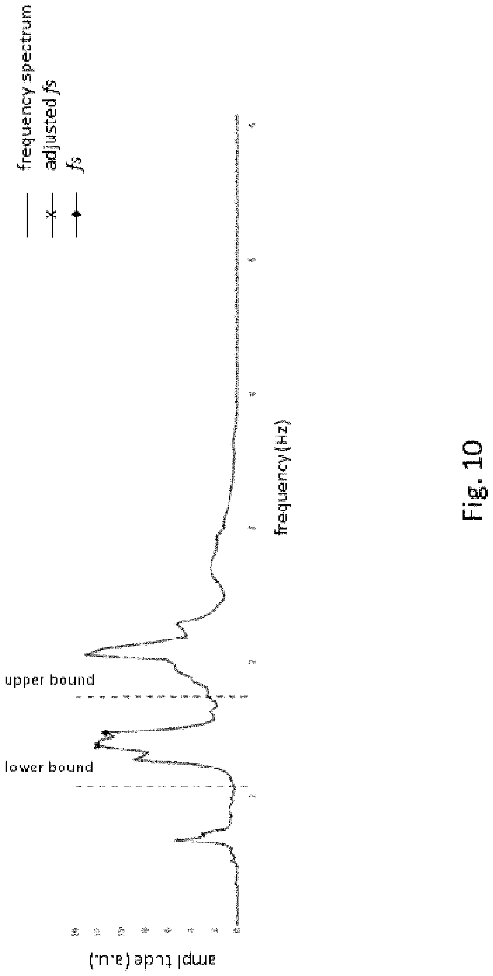

Subsequently, in order to maintain only frequencies for each stationary time step segment, a frequency spectrum (step **S532**) and corresponding main frequency *fs* for is determined (step SS31). This is illustrated schematically and for illustrative purposes for a single exemplary stationary time step segment in **Fig. 10**. The determined main frequency *fs* may further be adjusted based on the closest peak in the frequency spectrum, wherein the frequency of the closest peak is defined as adjusted *fs*. Based on the position of the adjusted *fs* in the frequency spectrum, upper bounds, *fsu*, and lower *bounds, fsl,* are determined (step SS33), wherein*fsu* and *fsl* may be defined at respective frequencies corresponding to local minima next to the peak. To maintain only the band of frequencies between *fsu* and *fsl* relevant for the type of motion to be detected and quantified, a zero-phase third-degree Butterworth bandpass filter is applied to the stationary time step segment of the time-domain data signal (step S534).

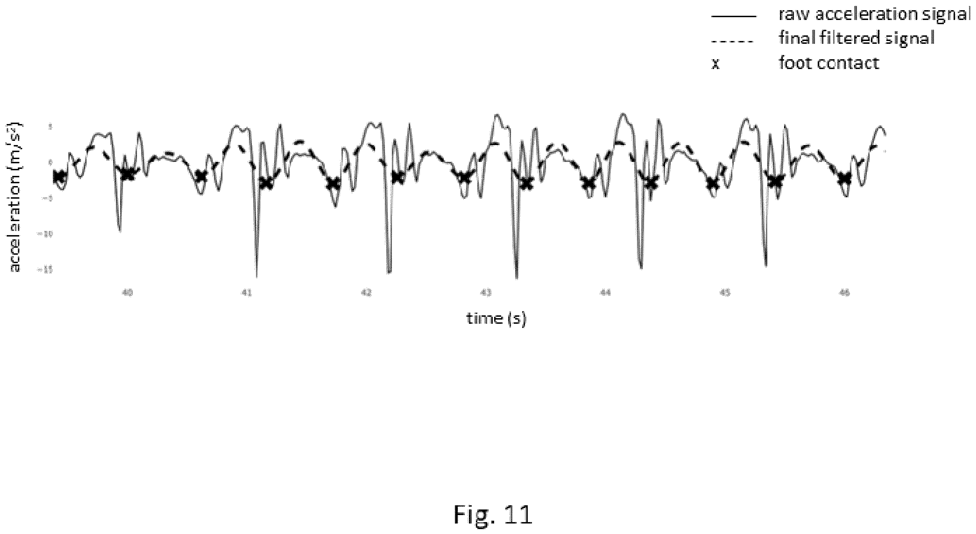

Motion data can subsequently be reconstructed using the plurality of filtered stationary time step segments of the time-domain data signal (step **S600**). Since stationary time step segments of the time-domain data signal are separated by time steps for which jumps in the ridge are determined, time step "gaps" between stationary time step segments may be reconstructed via interpolation to obtain final filtered reconstructed motion data (step **S610**). **Fig. 11** illustrates schematically the raw acceleration data, indicated by solid lines, overlaid with the final filtered reconstructed acceleration data, indicated by dashed lines, for the exemplary stationary time step segment.

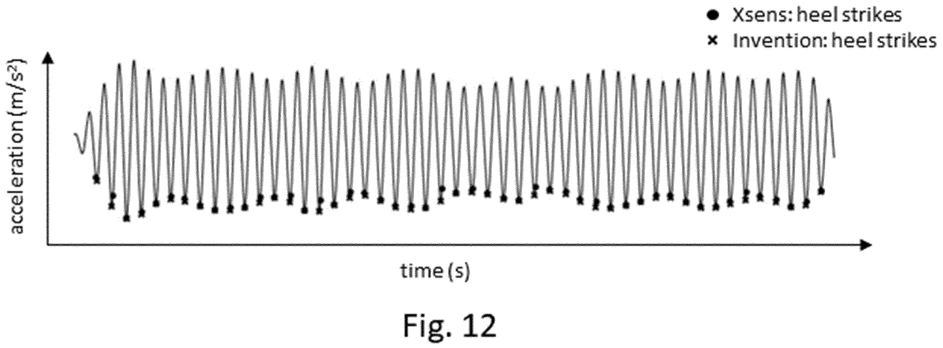

From the final filtered signal, a type of human motion - in this case gait - can be determined (step **S700**). As illustrated in Fig. 11, local extrema in the final filtered signal can be detected (step **S710**). In this case, first, valleys in the final filtered signal are detected (step **S711**), wherein a valley indicates a foot contact with the ground, and a step is indicated by pairs of consecutive valleys. To further reduce errors in determining gait, the expected maximum temporal distance between valleys is checked by applying heuristics based on the amplitude of extrema in the final filtered signal and based on motion-related frequency *fa* at a given time step (steps **S712** and **S713**). Based on the determined foot contacts as illustrated for the final filtered signal illustrated in **Fig. 12**, the individual's gait can be determined (step **S720**).

It is to be noted that, within the context of the present disclosure, a time step corresponds to the smallest time unit that may correspond to the temporal resolution of the raw motion data of time-domain data signal based on its respective sampling rate. For example, in case raw motion data or time-domain data signal is obtained with a sampling rate of 100 Hz, a time step may correspond to 0.01 s.

A method as described within the context of any one of the above embodiments may be performed by a single computing device or system. For example, raw motion data may be obtained via dedicated sensors provided with an individual, and subsequently communicated to a remote device, such as, e.g., a mobile, wearable, or stationary computing device or computing system, where some or all of the above-described steps for detecting and quantifying human motion are performed. As an example, the mobile device may be a smartphone, tablet, or laptop computer. As a further example, the computing system may be a distributed computing system or part of a distributed computing system, such as, e.g., a cloud system or cloud server. Additionally, or alternatively, some or all raw motion data may be obtained from sensors provided with a mobile or wearable device such as a smartphone or smartwatch provided with the individual, and subsequent steps for detecting and quantifying human motion may be performed by the smartphone or smartwatch.

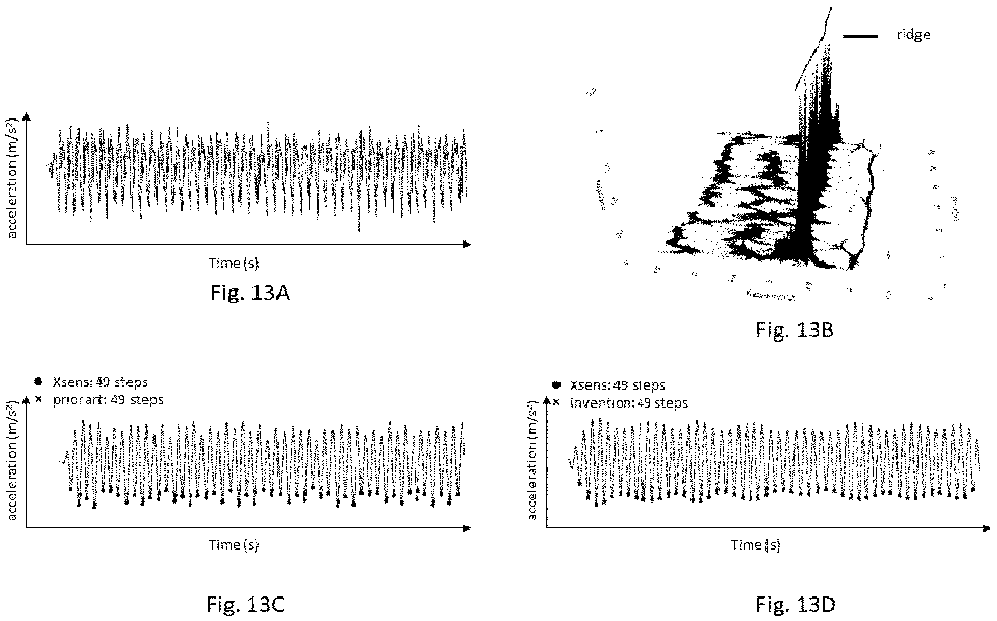

**Figs. 13A to 13C** illustrate a method according to embodiments of the disclosure in comparison to methods of the prior art for gait detection and quantification. **Fig. 13A** illustrates raw acceleration data obtained from IMU sensors of a first smartphone arranged in a front waist belt position of a healthy individual walking for 30 s with a regular cadence. Here, a method as described within the context of Fig. 3 was performed. **Fig. 13B** illustrates the ridge determined as described within the context of embodiments of Figs. 1 to 3. **Fig. 13C** illustrates the acceleration time-domain data signal obtained via a CWT-based method of the prior art [10] for step segmentation, while **Fig. 13D** illustrates the final filtered acceleration time-domain data signal obtained according to the present disclosure for step segmentation. Both the CWT-based method of the prior art and the method according to the present disclosure detected and quantified 49 steps of the individual during a predetermined time. To compare the performance of the CWT-based method of the prior art and of the present disclosure, the individual was provided with an 18-IMUs reference motion capture system (Xsens) to provide a ground truth, i.e., the actual number of steps taken by the individual. In the present case, both the CWT-based method of the prior art and methods of the present disclosure accurately detected the same number of steps as the Xsens system at 49 steps. Notably, the CWT-based method of the prior art performed well as it was designed for motion data obtained from a position near the individual's center of mass.

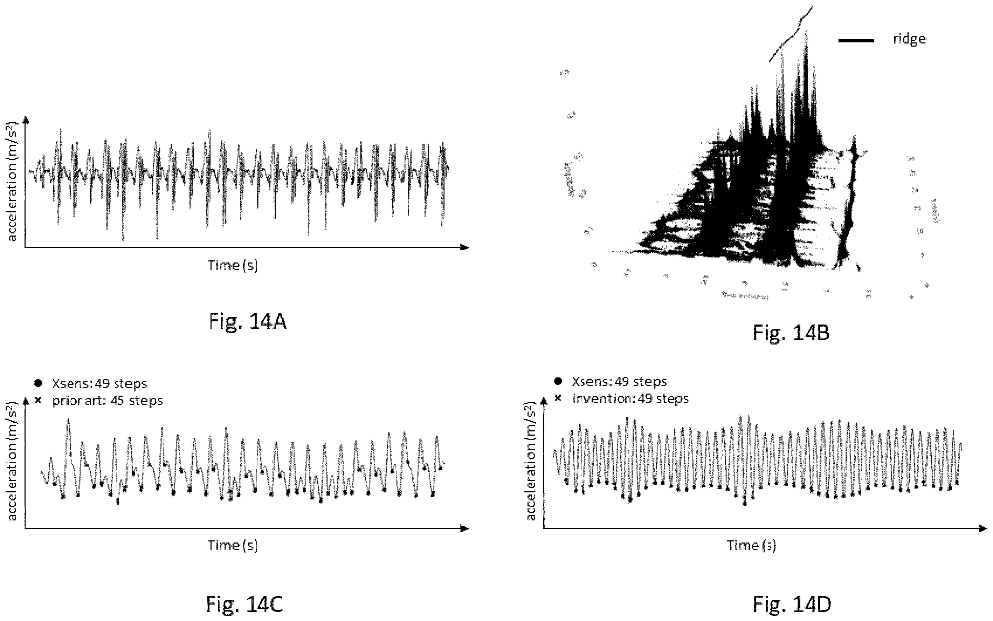

**Fig. 14A** illustrates raw acceleration data that was obtained at the same time and from the same individual as the raw acceleration data of Fig. 13A, with the difference that the raw acceleration data was obtained from IMU sensors from a second smartphone arranged in a pant front pocket position instead of a front waist belt position of the individual. The second smartphone was therefore arranged further away from the individual's center of mass than the first smartphone. Here, a method as described within the context of Fig. 3 was performed. **Fig. 14B** illustrates the correspondingly determined ridge in the TFR. **Fig. 14C** illustrates the acceleration time-domain data signal obtained via the CWT-based method of the prior art [10] for step segmentation, while **Fig. 14D** illustrates the final filtered acceleration time-domain data signal obtained according to the present disclosure for step segmentation. As can be seen, the CWT-based method of the prior art only determined 45 steps taken by the individual, while the method according to the present disclosure determined 49 steps taken by the individual. The method according to the present disclosure matched the number of steps determined via Xsens, and thus provides improved performance over the CWT-based method of the prior art even for sub-optimal placement of the smartphone.

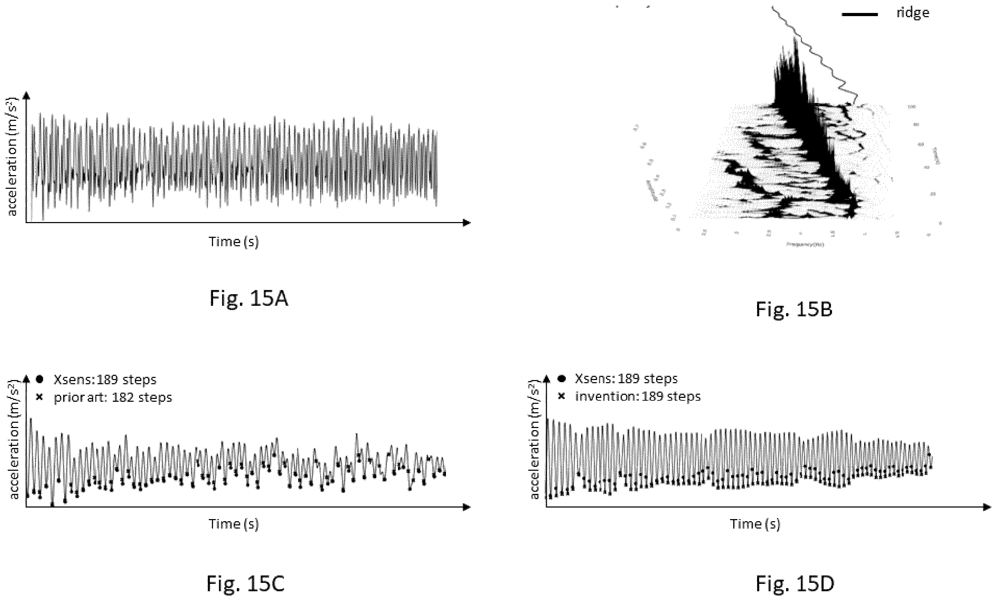

**Figs. 15A to 15D** illustrate a method according to embodiments of the disclosure in comparison to methods of the prior art for gait detection and quantification. **Fig. 15A** illustrates raw acceleration data obtained from IMU sensors of a first smartphone arranged in a front waist belt position of a healthy individual walking for 100s with varying cadence, wherein the individual progressively accelerated every 10s. Here, a method as described within the context of Fig. 3 was performed. **Fig. 15B** illustrates the correspondingly determined ridge. **Fig. 15C** illustrates the acceleration time-domain data signal obtained via the CWT-based method of the prior art [10] for step segmentation, while **Fig. 15D** illustrates the final filtered acceleration time-domain data signal obtained according to the present disclosure for step segmentation. As can be seen, the CWT-based method of the prior art only determined 182 steps taken by the individual, while the method according to the present disclosure determined 189 steps taken by the individual. The method according to the present disclosure matched the number of steps determined via Xsens and thus provides improved performance over the CWT method of the prior art. In contrast, the CWT method of the prior art determined 7 steps less than the actual number of steps taken.

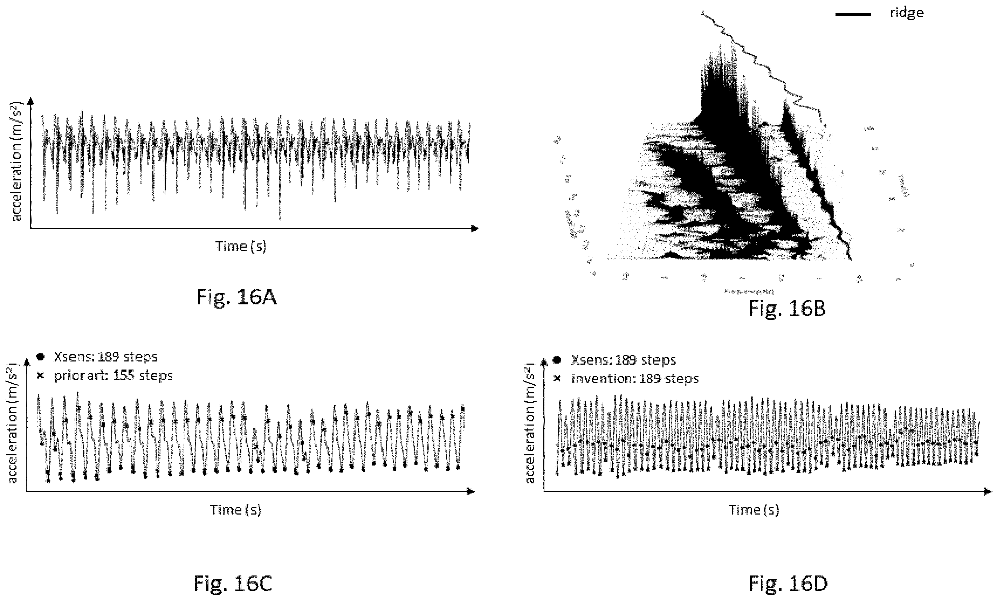

**Figs. 16A to 16D** illustrate a method according to embodiments of the disclosure in comparison to methods of the prior art for gait detection and quantification. **Fig. 1616A** illustrates raw acceleration data obtained at the same time as the raw acceleration data illustrated in Fig. 15A but from IMU sensors of a second smartphone arranged in a pants front pocket position of a healthy individual walking for 100s with varying cadence, wherein the individual progressively accelerated every 10s. Here, a method as described within the context of Fig. 3 was performed. **Fig. 16B** illustrates the correspondingly determined ridge. **Fig. 16C** illustrates the acceleration time-domain data signal obtained via the CWT-based method of the prior art [10] for step segmentation, while **Fig. 16D** illustrates the final filtered acceleration time-domain data signal obtained according to the present disclosure for step segmentation. As can be seen, the CWT-based method of the prior art only determined 155 steps taken by the individual, while the method according to the present disclosure determined 189 steps taken by the individual. The method according to the present disclosure matched the number of steps determined via Xsens and thus provides improved performance over the CWT method of the prior art. In contrast, the CWT method of the prior art determined 34 steps less than the actual number of steps taken.

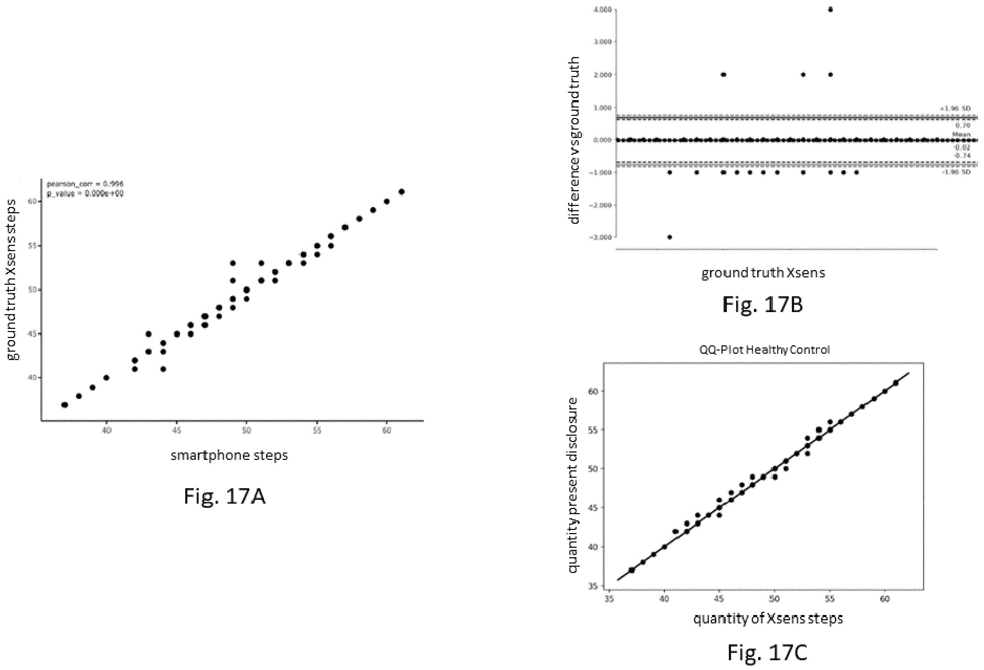

**Figs. 17Ato 17C** illustrate a comparison of methods of the present disclosure for gait detection for normal walking cadence in healthy individuals for data obtained from 385 cases. For each case, an individual walked for 30s with a normal cadence and was provided with Xsens. **Fig. 17A** illustrates a plot of ground truth steps determined via Xsens against steps determined from smartphone sensor according to methods of the present disclosure. **Fig. 17B** illustrates a Bland Altman plot representing differences in determined steps between Xsens and methods of the present disclosure. **Fig. 17C** illustrates a Q-Q-Plot comparing the steps determined according to methods of the present disclosure against steps determined via Xsens as the theoretical distribution. As evident, these plots show excellent correlation and level of agreement between the number of steps determined via Xsens and the number determined according to methods of the present disclosure.

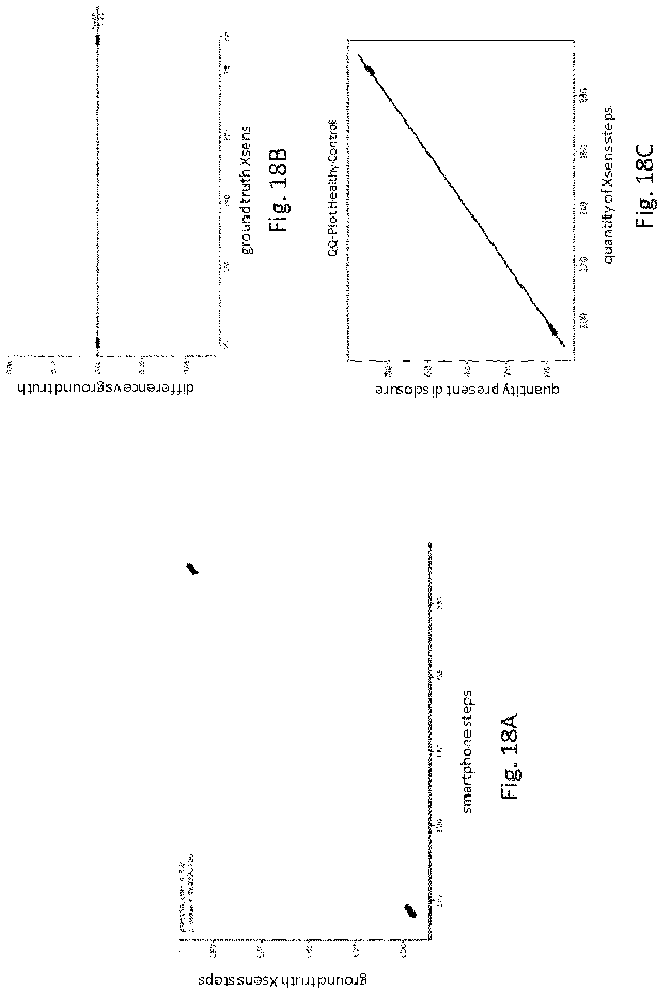

**Figs. 18Ato 18C** illustrate a comparison of methods of the present disclosure for gait detection for a variable walking cadence in healthy individuals for data obtained from 15 cases. For each case, an individual walked for 100s and was provided with Xsens. The individual progressively accelerated every 10 s. **Fig. 18A** illustrates a plot of ground truth steps determined via Xsens against steps determined from smartphone sensor according to methods of the present disclosure. **Fig. 18B** illustrates a Bland Altman plot representing differences in determined steps between Xsens and methods of the present disclosure. **Fig. 18C** illustrates a Q-Q-Plot comparing the steps determined according to methods of the present disclosure against steps determined via Xsens as the theoretical distribution. As evident, these plots show excellent correlation and level of agreement between the number of steps determined via Xsens and the number determined according to methods of the present disclosure.

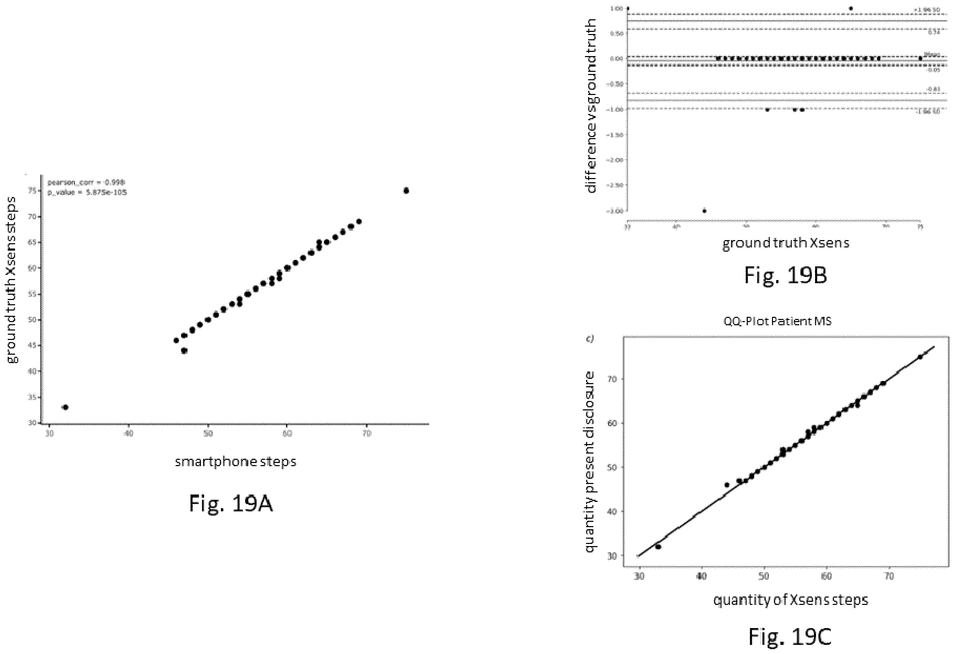

**Figs. 19 to 19C** illustrate a comparison of methods of the present disclosure for gait detection for regular walking cadence in individuals with multiple sclerosis for data obtained from 87 cases. For each case, an individual with multiple sclerosis walked at a regular cadence for 30s and was provided with Xsens. **Fig. 19A** illustrates a plot of ground truth steps determined via Xsens against steps determined from smartphone sensor according to methods of the present disclosure. **Fig. 19B** illustrates a Bland Altman plot representing differences in determined steps between Xsens and methods of the present disclosure. **Fig. 19C** illustrates a Q-Q-Plot comparing the steps determined according to methods of the present disclosure against steps determined via Xsens as the theoretical distribution. As evident, these plots show excellent correlation and level of agreement between the number of steps determined via Xsens and the number determined according to methods of the present disclosure.

While methods according to the present disclosure were described above within the context of Figs. 4 to 19C for gait detection, methods of the present disclosure can be utilized to detect other types of human motion.

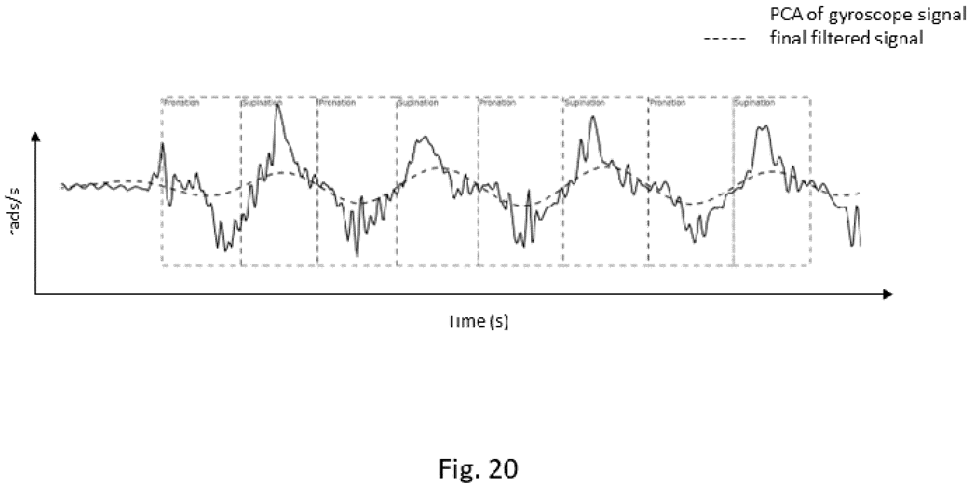

**Fig. 20** illustrates obtained raw motion data overlaid with a final filtered signal for detecting and quantifying pronation-supination to assess upper limb function, particularly focusing on motor control, coordination, and strength of the forearm and wrist muscles. The pronation-supination test can be used to evaluate individuals with neurological conditions or musculoskeletal issues affecting the forearm, wrist, and hand. For this, an individual holds a smartphone with one hand and alternately rotates the forearm for 10 seconds between two positions via pronation - Counterclockwise movement (CCW), and supination - clockwise movement (CW). For example, the two positions may be defined by the palm pointing downwards and the palm pointing upwards.

As the pronation-supination motion of the arm is distinct from gait, certain aspects of embodiments described above within the context of Figs. 4 to 19C are preferably adapted. For example, raw motion data may be obtained from gyroscopes of the smartphone (step **S100, S110).** It should be noted that the sensor is not limited to smartphone sensors and that any suitable gyroscope sensor can be used. For obtaining a time-domain data signal (step **S200),** pre-processing (step **S210)** is preferably performed by applying a Butterworth low-pass filter at 1 Hz (step **S212).** For aligning the raw motion data to a global reference frame (step **S211),** the main direction of the hand movement can be extracted by applying a principal component analysis (PCA) over the three-axial gyroscope signal. Determining the TFR (step **S300)** and determining the ridge (step **S400)** from the time-domain data signal can be performed via a simpler approach compared to the approach for detecting gait described above within the context of Figs. 4 to 19C. For example, an approach as proposed in [11] can be used. The more generic approach proposed in [11] determines a ridge by extracting the dominant frequency of each time step. This can suffice when the target motion of interest is simple and well defined, e.g., unidirectional motion of only one arm or one joint, and when the task is executed in a controlled and supervised setting, as such motion generally generates less signal noise and artifacts compared to unsupervised patterns of more complex movements such as gait. In the context of complex movements such as, e.g., gait, determining a ridge as described above within the context of Figs. 4 to 19C avoids undesired ridge values that would be produced by the approach proposed in [11]. Additionally, the segmentation of the hand/forearm motion in the final filtered signal (step S700) can be achieved by determining zero-crossing in the final filtered reconstruction motion data (step **S710)**.

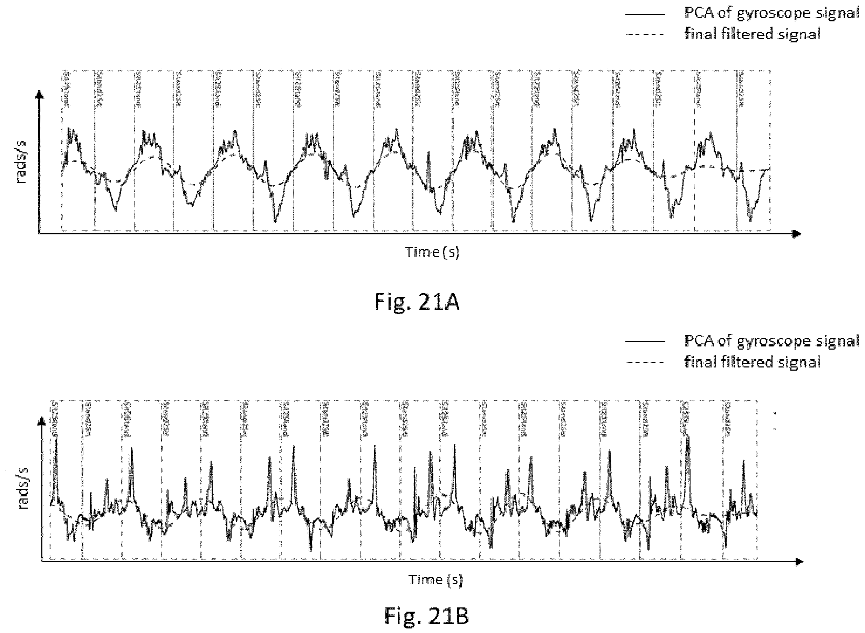

**Figs. 21A and 21B** illustrate obtained raw motion data overlaid with a final filtered signal for detecting and quantifying sit-stand transition motion to evaluate lower body strength, balance, functional mobility and fatigue. This test determines how efficiently an individual can move from a seated position to a standing position and vice versa. For this, an individual performs as many sit-stand transitions as possible during a time interval of 30 s. As the sit-stand-transition motion is distinct from gait, certain aspects of embodiments described above within the context of Figs. 4 to 19C are preferably adapted. For example, raw motion data can be obtained from gyroscopes and/or accelerometers of a smartphone (step S100, S110). It should be noted that the sensor is not limited to smartphone sensors and that any suitable gyroscope and/or accelerometer sensor can be used.

In the case illustrated in Fig. 21A, a smartphone was placed in the pants front pocket of an individual, and gyroscope raw motion data was obtained. In the case illustrated in Fig. 21B, raw accelerometer motion data was obtained from the same smartphone. In both cases, for obtaining a time-domain data signal (step **S200),** pre-processing (step **S210)** is preferably performed by applying Butterworth low-pass filter at 1 Hz (step **S212).** Determining the TFR (step **S300)** and determining the ridge (step **S400)** from the time-domain data signal can be performed via a simpler approach compared to the approach for detecting gait described above within the context of Figs. 4 to 19C. For example, an approach as proposed in [11] can be used. The segmentation of the sit-stand transition motion in the final filtered signal (step **S700)** can be achieved by determining zero-crossing in the final filtered reconstruction motion data (step **S710)** when gyroscope raw motion data is used. Points where the angular speed is zero correspond to changes in the direction of motion and thus reflect transitions between a stand-to-sit and a sit-to-stand motion. Additionally, or alternatively, the segmentation of the sit-stand transition motion in the final filtered signal (step **S700)** can be achieved by determining peaks in the final filtered reconstructed motion data (step **S710)** when accelerometer raw motion data is used. Here, each positive or negative peak corresponds to a change in the direction of motion and thus reflects transitions between a stand-to-sit and a sit-to-stand motion.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any one of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

A processor can be a microprocessor, but in the alternative, the processor can be any conventional processor, controller, or state machine. A processor can also be implemented as a combination of computing devices, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein but is to be accorded the widest scope consistent with the novel features and principles disclosed herein, as recited in the claims below.

### List of cited references

[1] Ridao-Fernández C, Pinero-Pinto E, Chamorro-Moriana G. Observational Gait Assessment Scales in Patients with Walking Disorders: Systematic Review. Biomed Res Int. 2019 Oct 31;2019:2085039. doi: 10.1155/2019/2085039. PMID: 31781597; PMCID: PMC6875351.
[2] Givon, U., Zeilig, G., & Achiron, A. (2009). Gait analysis in multiple sclerosis: characterization of temporal-spatial parameters using GAITRite functional ambulation system. Gait & posture, 29(1), 138-142.
[3] Pilutti, L. A., Dlugonski, D., Sandroff, B. M., Suh, Y., Pula, J. H., Sosnoff, J. J., & Motl, R. W. (2013). Gait and six-minute walk performance in persons with multiple sclerosis. Journal of the neurological sciences, 334(1-2), 72-76.
[4] Fuller, D., Colwell, E., Low, J., Orychock, K., Tobin, M. A., Simango, B., Buote, R., Van Heerden, D., Luan, H., Cullen, K., Slade, L., & Taylor, N. (2020). Reliability and validity of commercially available wearable devices for measuring steps, energy expenditure, and heart rate: Systematic review. JMIR mHealth and uHealth, 8(9), e18694.
[5] Little, M. A. (2021). Smartphones for remote symptom monitoring of Parkinson's disease. Journal of Parkinson's Disease, 11(s1), S49-S53.
[6] Bo, F., Yerebakan, M., Dai, Y., Wang, W., Li, J., Hu, B., & Gao, S. (2022, June). IMU-based monitoring for assistive diagnosis and management of IoHT: a review. In Healthcare (Vol. 10, No. 7, p. 1210). MDPI.
[7] Nguyen, L. V., & La, H. M. (2016). Real-time human foot motion localization algorithm with dynamic speed. IEEE Transactions on Human-Machine Systems, 46(6), 822-833.
[8] Negi, S., Purwar, A., Ojha, R., & Vashishtha, A. (2021). FSR and IMU sensors-based human gait phase detection and its correlation with EMG signal for different terrain walk. Sensor Review, 41(3), 235-245
[9] Romijnders, R., Warmerdam, E., Hansen, C., Schmidt, G., & Maetzler, W. (2022). A deep learning approach for gait event detection from a single shank-worn IMU: Validation in healthy and neurological cohorts. Sensors, 22(10), 3859.
[10] McCamley, J., Donati, M., Grimpampi, E., & Mazzà, C. (2012). An enhanced estimate of initial contact and final contact instants of time using lower trunk inertial sensor data. Gait & Posture, 36(2), 316-318.
[11] latsenko, D., McClintock, P. V. E., & Stefanovska, A. (2016). On the extraction of instantaneous frequencies from ridges in time-frequency representations of signals. Digital Signal Processing, 42, 1-26.

## Claims

1. Method for motion detection and quantification, the method comprising:
a) obtaining a time-domain data signal based on raw motion data;
b) determining a time-frequency representation, TFR, of the data signal;
c) determining a ridge in the TFR, wherein the ridge corresponds to locations of maximum energy or maximum amplitude in the TFR and comprises a motion-related frequency, *fa,* per each time step of the time-domain data signal;
d) splitting the time-domain data signal into one or more stationary time step segments based on the determined ridge;
e) reconstructing motion data based on the one or more stationary time step segments of the data signal; and
f) determining and quantifying the motion data based on temporal characteristics of the reconstructed motion data.

2. The method according to claim 1, wherein, in a), the raw motion data is obtained from one or more inertial measurement unit, IMU, sensors which preferably comprise one or more of:
an accelerometer; and/or
a gyroscope.

3. The method according to any of claims 1 to 2, wherein, in c), determining the ridge comprises:
splitting the time-domain data signal and the TFR into a plurality of overlapping time step windows, and
obtaining a dominant frequency, *ff,* for each time step window.

4. The method according to claim 3, wherein obtaining *ff* for each time step window comprises:
determining a main frequency, *f0*, using autocorrelation on the respective data signal time step window, and
correcting *f0* by determining the closest dominant frequency in the frequency spectrum of the corresponding TFR time step window.

5. The method according to claim 4, wherein in c), *fa* is obtained based on the obtained *ff* values of the time step windows.

6. The method according to claim 5, wherein in c), for each respective time step, *fa* is obtained by averaging the *ff* values of all the time step windows that contain the respective time step.

7. The method according to any one of claims 3 to 6, wherein each time step window overlaps adjacent time step windows by more than 70%, preferably by more than 80%, more preferably by more than 90%, even more preferably by more than 95% of the window time step size.

8. The method according to any one of claims 1 to 7, wherein in d), splitting the time-domain data signal into stationary time step segments comprises:
determining stationary time step segments based on one or more jumps in the ridge that correspond to changes of the ridge that exceed a predetermined frequency threshold, and
splitting the motion data into stationary time step segments separated by respective time steps where the one or more jumps in the ridge are determined.

9. The method according to claim 8, wherein the predetermined frequency threshold is in a range from 0.05 Hz to 0.2 Hz, preferably from 0.07 to 0.13 Hz, most preferably about 0.1 Hz.

10. The method according to any one of claims 1 to 9, wherein in e), reconstructing the motion data comprises, for each stationary time step segment:
determining a mean main frequency, *fs*, of a respective stationary time step segment by averaging *fa* values of the respective segment of the ridge that corresponds to the time steps of the respective stationary time step segment;
determining a frequency spectrum of the respective segment of the TFR;
determining upper and lower bounds for the band frequency around *fs*; and
filtering each respective stationary time step segment using a bandpass filter.

11. The method according to any one of claims 9 to 10, wherein in e), reconstructing the motion data further comprises reconstructing a final filtered signal based on the respective filtered stationary time step segments.

12. The method according to the preceding claim, wherein in e), determining and quantifying the motion data comprises determining motion events based on one or more of:
local extrema in the reconstructed motion data,
zero-crossing in the reconstructed motion data,
an amplitude of the reconstructed motion data, and/or
a distance between local extrema based on frequencies comprised by the ridge.

13. An apparatus comprising at least one processor configured to perform a method according to any one of the preceding claims.

14. Computer program product comprising instructions that, when executed by an apparatus comprising at least one processor, cause the apparatus to perform a method according to any one of claims 1 to 12.

15. Computer-readable storage medium comprising the computer program product according to the preceding claim.

## Amended claims

### Amended claims in accordance with Rule 137(2) EPC.

1. Method for motion detection and quantification, the method comprising:
a) obtaining (200) a time-domain data signal based on raw motion data;
b) determining (300) a time-frequency representation, TFR, of the data signal;
c) determining (400) a ridge in the TFR, wherein the ridge corresponds to locations of maximum energy or maximum amplitude in the TFR and comprises a motion-related frequency, *fa*, per each time step of the time-domain data signal;
d) splitting (500) the time-domain data signal into one or more stationary time step segments, in which one or more statistical properties of the time-domain data signal do not substantially change, based on the determined ridge, wherein splitting the time-domain data signal into stationary time step segments comprises:
determining stationary time step segments based on one or more jumps in the ridge that correspond to changes of the ridge that exceed a predetermined frequency threshold, and
splitting the motion data into stationary time step segments separated by respective time steps where the one or more jumps in the ridge are determined;
e) reconstructing (600) motion data based on the one or more stationary time step segments of the data signal, wherein reconstructing the motion data comprises, for each stationary time step segment:
determining a mean main frequency, fs, of the stationary time step segment by averaging *fa* values of the respective segment of the ridge;
determining a frequency spectrum of the respective segment of the TFR;
determining, on the frequency spectrum of the respective segment of the TFR, upper and lower bounds for the band frequency around fs; and
filtering the respective stationary time step segment using a bandpass filter; and
f) determining and quantifying (700) the motion data based on temporal characteristics of the reconstructed motion data.

2. The method according to claim 1, wherein, in a), the raw motion data is obtained from one or more inertial measurement unit, IMU, sensors which preferably comprise one or more of:
an accelerometer; and/or
a gyroscope.

3. The method according to any of claims 1 to 2, wherein, in c), determining the ridge comprises:
splitting the time-domain data signal and the TFR into a plurality of overlapping time step windows, and
obtaining a dominant frequency, *ff*, for each time step window.

4. The method according to claim 3, wherein obtaining *ff* for each time step window comprises:
determining a main frequency, *f0,* using autocorrelation on the respective data signal time step window, and
correcting *f0* by determining the closest dominant frequency in the frequency spectrum of the corresponding TFR time step window.

5. The method according to claim 4, wherein in c), *fa* is obtained based on the obtained *ff* values of the time step windows.

6. The method according to claim 5, wherein in c), for each respective time step, *fa* is obtained by averaging the *ff* values of all the time step windows that contain the respective time step.

7. The method according to any one of claims 3 to 6, wherein each time step window overlaps adjacent time step windows by more than 70%, preferably by more than 80%, more preferably by more than 90%, even more preferably by more than 95% of the window time step size.

8. The method according to claim 1, wherein the predetermined frequency threshold is in a range from 0.05 Hz to 0.2 Hz, preferably from 0.07 to 0.13 Hz, most preferably about 0.1 Hz.

9. The method according to claim 1, wherein in e), reconstructing the motion data further comprises reconstructing a final filtered signal based on the respective filtered stationary time step segments.

10. The method according to the preceding claim, wherein in f), determining and quantifying the motion data comprises determining motion events based on one or more of:
local extrema in the reconstructed motion data,
zero-crossing in the reconstructed motion data,
an amplitude of the reconstructed motion data, and/or
a distance between local extrema based on frequencies comprised by the ridge.

11. An apparatus comprising at least one processor configured to perform a method according to any one of the preceding claims.

12. Computer program product comprising instructions that, when executed by an apparatus comprising at least one processor, cause the apparatus to perform a method according to any one of claims 1 to 10.

13. Computer-readable storage medium comprising the computer program product according to the preceding claim.
